(19) Europäisches Patentamt
European Patent Office
Office européen des brevets

(11) Veröffentlichungsnummer: **0 298 919**
**A2**

(12) # EUROPÄISCHE PATENTANMELDUNG

(21) Anmeldenummer: **88810453.6**

(22) Anmeldetag: **01.07.88**

(51) Int. Cl.[4]: **C 07 C 93/26**
C 07 C 91/46, C 07 D 217/04, C 07 D 215/06, C 07 D 211/26, C 07 F 9/40, C 07 C 97/22, C 07 D 295/10, A 61 K 31/13, A 61 K 31/215, A 61 K 31/47

(30) Priorität: **10.07.87 US 71956**

(43) Veröffentlichungstag der Anmeldung: **11.01.89 Patentblatt 89/02**

(84) Benannte Vertragsstaaten: **AT BE CH DE ES FR GB GR IT LI LU NL SE**

(71) Anmelder: **CIBA-GEIGY AG**
**Klybeckstrasse 141**
**CH-4002 Basel (CH)**

(72) Erfinder: **Wasley, Jan W.F.**
**55 Floral Street**
**Chatham New Jersey 07928 (US)**

(54) **4-Amino-substituierte 1,2-Dihydroxynaphthalin-Derivate.**

(57) Die Erfindung betrifft neue Verbindungen der Formel I

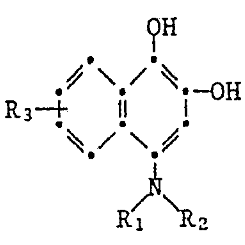

(I)

worin $R_1$ unsubstituiertes oder durch Niederalkyl substituiertes $C_3$-$C_7$-Cycloalkyl, unsubstituiertes oder durch Niederalkyl substituiertes $C_7$-oder $C_8$-Bicycloalkyl, unsubstituiertes oder durch Niederalkyl substituiertes Adamantyl, 4-Piperdinyl oder N-Niederalkyl oder Aryl-niederalkyl substituiertes Piperidinyl, 1- oder 2-Indanyl, 1- oder 2-Tetrahydronaphthyl, 1- oder 2-Perhydroindanyl, 1- oder 2-Perhydronaphthyl bedeutet; $R_2$ Wasserstoff, Niederalkyl, $C_3$-$C_7$-Cycloalkyl, Aryl-niederalkyl $C_3$-$C_7$-Cycloalkyl-niederalkyl, Niederalkanoyl oder Aryl-niederalkanoyl darstellt; oder $R_1$ und $R_2$, zusammen mit dem Stickstoffatom, an das sie gebunden sind, für Pyrrolidino, Piperidino, Perhydroazepino, Morpholino, Thiomorpholino, Tetrahydro- oder Perhydro(isochinolinyl oder chinolinyl), Dihydro- oder Perhydro-indolyl, Piperazino oder N-Niederalkyl-piperazino stehen; $R_3$ Wasserstoff, Niederalkyl, Halogen oder Niederalkoxy bedeutet; pharmazeutisch annehmbare Ester- und Etherderivate davon; und pharmazeutisch verwendbare Säureadditionssalze der basischen Verbindungen der Formel I und ihrer Derivate. Diese Verbindungen wie auch gewisse Zwischenprodukte, aus denen sie hergestellt werden können, wirken als 5-Lipoxygenase-Hemmer.

EP 0 298 919 A2

**Beschreibung**

**4-Amino-substituierte 1,2-Dihydroxynaphthalin-Derivate**

Die vorliegende Erfindung betrifft neue 4-Amino-substituierte 1,2-Dihydroxynaphthalin-Derivate und Zwischenprodukte, welche als 5-Lipoxygenase-Hemmer verwendbar sind, Verfahren zur Herstellung dieser Verbindungen, pharmazeutische Zusammensetzungen, welche diese Verbindungen enthalten, und Methoden zur Hemmung von 5-Lipoxygenase und zur Behandlung von Krankheiten in Säugern, welche durch 5-Lipoxygenasehemmung regulierbar sind, durch Anwendung der genannten Verbindungen oder einer pharmazeutischen Zusammensetzung enthaltend die erfindungsgemässen Verbindungen.

Die Erfindung betrifft neue Verbindungen der Formel I

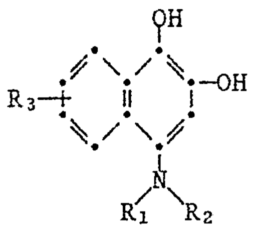

(I)

worin $R_1$ unsubstituiertes oder durch Niederalkyl substituiertes $C_3$-$C_7$-Cycloalkyl, unsubstituiertes oder durch Niederalkyl substituiertes $C_7$-oder $C_8$-Bicycloalkyl, unsubstituiertes oder durch Niederalkyl substituiertes Adamantyl, 4-Piperidinyl oder N-Niederalkyl oder Aryl-niederalkyl substituiertes Piperidinyl, 1- oder 2-Indanyl, 1- oder 2-Tetrahydronapthyl, 1- oder 2-Perhydroindanyl, 1- oder 2-Perhydronaphthyl bedeutet; $R_2$ Wasserstoff, Niederalkyl, $C_3$-$C_7$-Cycloalkyl, Aryl-niederalkyl, $C_3$-$C_7$-Cycloalkyl-niederalkyl, Niederalkanoyl oder Aryl-niederalkanoyl darstellt; oder $R_1$ und $R_2$, zusammen mit dem Stickstoffatom, an das sie gebunden sind, für Pyrrolidino, Piperidino, Perhydroazepino, Morpholino, Thiomorpholino, Tetrahydro- oder Perhydro-(isochinolinyl oder chinolinyl), Dihydro- oder Perhydro-indolyl, Piperazino oder N-Niederalkyl-piperazino stehen; $R_3$ Wasserstoff, Niederalkyl, Halogen oder Niederalkoxy bedeutet; pharmazeutisch annehmbare Ester- und Etherderivate davon; und pharmazeutisch verwendbare Säureadditionssalze der basischen Verbindungen der Formel I und ihrer Derivate.

Pharmazeutisch annehmbare Esterderivate sind Verbindungen der Formel I, in denen eine oder beide Hydroxygruppen in Form eines pharmazeutisch annehmbaren Esters verestert sind; insbesondere zu Arzneimitteln verarbeitbare Ester, die z.B. durch Solvolyse unter physiologischen Bedingungen in Verbindungen der Formel I mit freien Hydroxygruppen umwandelbar sind.

Bevorzugt für die zu Arzneimitteln verarbeitbaren pharmazeutisch annehmbaren Ester sind Niederalkancarbonsäureester, Niederalkoxy-niederalkancarbonsäureester, Arylcarbonsäureester, Arylniederalkancarbonsäureester, Carbamid- oder N-Mono- oder N,N-Di-(niederalkyl, Aryl- oder Arylniederalkyl)-carbamidsäureester (Carbamate); Phosphorsäure oder O,O-Di-(niederalkyl, Aryl oder Aryl-niederalkyl)-phosphorsäureester; Kohlensäure- oder O-(Niederalkyl, Aryl oder Arylniederalkyl)-kohlensäure-ester; die cyclischen Kohlensäureester, in welchen beide Ring-Hydroxygruppen in Form eines cyclischen Carbonatester-Derivates verestert sind, oder cyclische Phosphorsäureester, in welchen beide Ring-Hydroxygruppen in Form eines cyclischen Phosphatester-Derivates verestert sind.

Pharmazeutisch annehmbare Etherderivate, in denen eine oder beide Hydroxygruppen als Ether derivatisiert sind, sind insbesondere Mono- oder Di-niederalkyl- oder Aryl-niederalkyl-ether, oder cyclische Nieder-alkylen-ether (in denen beide vicinal stehende Hydroxygruppen in Form von Niederalkylendioxygruppen verethert sind).

Bevorzugt für die zu Arzneimitteln verarbeitbaren pharmazeutisch annehmbaren Ester sind geradkettige oder verzweigte Niederalkancarbonsäureester, z.B. die Essig-, Isobutyr-, Pivalinsäureester; Niederalkoxyniederalkancarbonsäureester, z.B. die Methoxyessig-, 3-Ethoxypropionsäureester; Arylcarbonsäureester, z.B. Benzoe-, Nicotinsäureester; Carbamid- und N-Mono- oder N,N-Di-Niederalkyl oder Arylcarbamidsäureester (Carbamate), z.B. die N-Mono-oder N,N-Di-Ethylcarbamid- oder N-Mono- oder N, N-Di-Methylcarbamidsäureester.

Die Erfindung betrifft insbesondere Verbindungen der Formel II

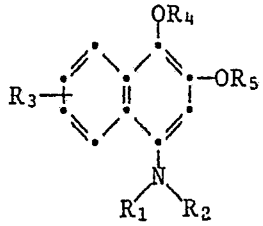

(II)

worin $R_1$ $C_5$-$C_7$-Cycloalkyl, Bicyclo[2.2.1]heptyl, Adamantyl, 1- oder 2-Indanyl oder Perhydro-indanyl, oder 1- oder 2-Tetrahydro- oder Perhydro-naphthyl bedeutet, $R_2$ Wasserstoff, Niederalkyl, Arylniederalkyl, $C_5$-$C_7$-Cycloalkylniederalkyl, Niederalkanoyl oder Arylniederalkanoyl darstellt, $R_3$ Wasserstoff, Niederalkyl oder Halogen ist, $R_4$ und $R_5$ unabhängig voneinander Wasserstoff, Niederalkanoyl, Aroyl, Niederalkyl, N-Arylcarbamoyl, N-Mono- oder N, N-Di-Alkylcarbamoyl, oder O,O-Di-niederalkylphosphonyl, oder $R_4$ und $R_5$ zusammen Carbonyl bedeuten; und pharmazeutisch annehmbare Säureadditionssalze davon, vorausgesesetzt, dass $R_2$ Wasserstoff, Niederalkyl, Arylniederalkyl oder $C_5$-$C_7$-Cycloalkylniederalkyl darstellt.

Die Erfindung betrifft insbesondere Verbindungen der Formel II, worin $R_1$ Cyclopentyl, Cyclohexyl, Cycloheptyl, 2-Norbornyl, 1- oder 2-Adamantyl, 1- oder 2-Indanyl oder 1- oder 2-Perhydro-indanyl bedeutet, $R_2$ Wasserstoff, $C_1$-$C_4$-Alkyl oder Aryl-$C_1$-$C_4$-alkyl ist, $R_3$ Wasserstoff, $C_1$-$C_4$-Alkyl oder Halogen bedeutet, $R_4$ Wasserstoff, $C_2$-$C_4$-Alkanoyl, N-Arylcarbamoyl, N-Mono-oder N,N-Di-$C_1$-$C_4$-Alkylcarbamoyl, und $R_5$ Wasserstoff, $C_1$-$C_4$-Alkyl, $C_2$-$C_4$-Alkanoyl, N-Arylcarbamoyl, N-Mono- oder N,N-Di-$C_1$-$C_4$-Alkylcarbamoyl oder O,O-Di-$C_1$-$C_4$-Alkylphosphonyl bedeuten; und pharmazeutisch annehmbare Säureadditionssalze davon.

Bevorzugt sind Verbindungen der Formel II, worin $R_1$ Cyclopentyl, Cyclohexyl oder Cycloheptyl, $R_2$ Niederalkyl, $R_3$ Wasserstoff, $R_4$ $C_2$-$C_4$-Alkanoyl, und $R_5$ $C_2$-$C_4$-Alkanoyl bedeuten; und pharmazeutisch annehmbare Säureadditionssalze davon.

Besonders bevorzugt sind Verbindungen der Formel II, worin $R_1$ Cyclohexyl, $R_2$ $C_1$-$C_4$-Alkyl, $R_3$ Wasserstoff, $R_4$ und $R_5$ $C_2$-$C_4$-Alkanoyl bedeuten; und pharmazeutisch annehmbare Säureadditionssalze davon.

Besonders bevorzugt sind Verbindungen der Formel II, worin $R_1$ und $R_2$ zusammen mit dem Stickstoffatom, an welches sie gebunden sind, für Pyrrolidino, Piperidino, Perhydroazepino, Morpholino, Thiomorpholino, Piperazino oder N-Niederalkyl-piperazino, Tetrahydro- oder Perhydro-isochinolinyl, Tetrahydro-oder Perhydro-chinolinyl, Dihydro- oder Perhydroindolinyl stehen, $R_3$ Wasserstoff, Niederalkyl, oder Halogen ist, $R_4$ und $R_5$ unabhängig voneinander Wasserstoff, Niederalkanoyl, Aroyl, Niederalkyl, N-Arylcarbamoyl, N-Mono- oder N,N-Dialkylcarbamoyl oder O,O-Di-Niederalkylphosphonyl, oder $R_4$ und $R_5$ zusammen Carbamoyl darstellen; und pharmazeutisch annehmbare Säureadditionssalze davon.

Insbesondere bevorzugt sind Verbindungen der Formel II, worin $R_1$ und $R_2$ zusammen mit dem Stickstoffatom, an welches sie gebunden sind, für Tetrahydro- oder Perhydro-isochinolinyl, Tetrahydro- oder Perhydrochinolinyl, Dihydro- oder Perhydro-indolyl stehen, $R_3$ Wasserstoff, $R_4$ Wasserstoff, $C_2$-$C_4$-Alkanoyl, N-Arylcarbamoyl, N-Mono- oder N,N-Di-$C_1$-$C_4$-Alkylcarbamoyl, und $R_5$ Wasserstoff, $C_1$-$C_4$-Alkyl, $C_2$-$C_4$-Alkanoyl, N-Arylcarbamoyl, N-Mono- oder N,N-Di-Alkylcarbamoyl, oder O,O-Di-$C_1$-$C_4$-Alkyl phosphonyl bedeuten; und pharmazeutisch annehmbare Säureadditionssalze davon.

Am bevorzugtesten sind Verbindungen der Formel II, worin $R_1$ und $R_2$ zusammen mit dem Stickstoffatom, an welches sie gebunden sind, für Tetrahydro- oder Perhydro-chinolinyl, Tetrahydroisochinolinyl oder Perhydroisochinolinyl stehen, $R_3$ Wasserstoff ist, $R_4$ und $R_5$ $C_2$-$C_4$-Alkanoyl bedeuten; und pharmazeutisch annehmbare Säureadditionssalze davon.

Die allgemeinen Definitionen in der Beschreibung dieser Erfindung haben die folgenden Bedeutungen:

Der weiter vorn und im folgenden verwendete Ausdruck "Nieder" bedeutet, dass so bezeichnete organische Reste oder Verbindungen bis einschliesslich 7 und vorzugsweise bis einschliesslich 4 und insbesondere 1 oder 2 Kohlenstoffatome enthalten.

Niederalkyl enthält vorzugsweise 1-4 C-Atome und ist beispielsweise Ethyl, Propyl, Butyl oder insbesondere Methyl.

Niederalkoxy enthält vorzugsweise 1-4 C-Atome und ist bespielsweise Ethoxy, Propoxy, Isopropoxy oder insbesondere Methoxy.

Niederalkanoyl bedeutet insbesondere $C_2$-$C_4$-Alkanoyl, wie Acetyl oder Propionyl.

Halogen bedeutet insbesondere Chlor oder Fluor, kann jedoch für Brom oder Jod stehen.

Ein Aryl-Radikal (Ar) bedeutet insbesondere ein carbocyclisches Radikal.

Ein carbocyclisches Aryl-Radikal bedeutet insbesondere Phenyl oder Phenyl substituiert durch 1 oder 2 Substituenten, wie Niederalkyl, Niederalkoxy, Halogen, Cyano und Trifluormethyl; oder 1- oder 2-Naphthyl.

Aroyl bedeutet insbesondere Benzoyl oder Benzoyl substituiert durch 1 oder 2 Substituenten, wie Niederalkyl, Niederalkoxy, Halogen, Cyano, Trifluormethyl; oder 1- oder 2-Naphthoyl.

Bicycloalkyl bedeutet insbesondere unsubstituiertes oder durch Niederalkyl substituiertes Bicyclo[2.2.1]heptyl, wie Bornyl, Neobornyl, Isobornyl, Norbornyl, z.B. 2-Norbornyl, insbesondere 2-Exo-norbornyl.

Tricycloalkyl bedeutet insbesondere unsubstituiertes oder durch Niederalkyl substituiertes Adamantyl,

insbesondere 1- oder 2-Adamantyl.

Pharmazeutisch annehmbare Salze von den basischen erfindungsgemässen Verbindungen sind Säureadditionssalze, vorzugsweise therapeutisch verwendbare Salze von anorganischen oder organischen Säuren; z.B. starken Mineralsäuren, wie Halogenwasserstoffsäuren, z.B. Chlorwasserstoff- oder Bromwasserstoffsäure, Schwefel-, Phosphor- oder Salpetersäure; aliphatischen oder aromatischen Carbonsäuren oder Sulfonsäuren, z.B. Ameisen-, Essig-, Propion-, Bernstein-, Glycol-, Milch-, Apfel-, Wein-, Glucon-, Zitronen-, Malein-, Fumar-, Brenztrauben-, Phenylessig-, Benzoe-, 4-Aminobenzoe-, Anthranil-, 4-Hydroxybenzoe-, Salicyl-, 4-Aminosalicyl-, Pamoa-, Nicotin-, Methansulfon-, Ethansulfon-, Hydroxyethansulfon-, Benzolsulfon-, p-Toluolsulfon-, Naphthalinsulfon-, Sulfanil-, Cyclohexylsulfamin- oder Ascorbinsäure.

Die neuen erfindungsgemässen Verbindungen zeigen wertvolle pharmakologische Eigenschaften und sind insbesondere als selektive 5-Lipoxygenase-Hemmer wirksam und können bei der Behandlung von inflammatorischen und allergischen Krankheiten, wie z.B. Lungenallergie, Asthma, allergischen oder inflammatorischen Krankheiten des Auges, allergischen oder inflammatorischen Krankheiten der Haut, wie z.B. Psoriasis; zur Behandlung von rheumatischen Krankheiten, wie z.B. rheumatischen Arthritis; und auch zur Behandlung von ischämischen Krankheiten, wie z.B. Myocardialinfarkt und Cerebralischämie, verwendet werden.

Die genannten Eigenschaften lassen sich in in vitro- und in vivo-Versuchen, vorzugsweise bei Säugetieren, z.B. Ratten, Hunden, Meerschweinchen, Kaninchen oder auch an entnommenen isolierten Organen und Gewebeproben, und Enzympräparaten davon, wie Zellen und Flüssigkeiten isoliert aus dem Blut von Säugern, inklusive von menschen, demonstrieren.

Die erfindungsgemässen Verbindungen können in vitro in Form von Lösungen, z.B. wässrigen Lösungen, und in vivo entweder enteral oder parenteral, vorzugsweise oral, z.B. als Suspension oder wässrige Lösung, appliziert werden. Der Dosisbereich in vitro kann zwischen $10^{-4}$ und $10^{-9}$ molarer Konzentration betragen. Der Dosisbereich in vivo kann zwischen 0,01 und 25 mg/kg in Abhängigkeit von dem Verarbreichungsweg betragen.

5-HETE und andere Leukotrienprodukte werden aus Arachidonsäure mittels des Enzyms 5-Lipoxygenase gebildet. Leukotriene (LTs) $B_4$, $C_4$, $D_4$ und $E_4$ bilden eine Gruppe von Mediatoren mit starken Leukozyt-chemoattractanten, Kontraktion von glatter Muskulatur und vaskuläre Permeabilität unterstützenden Eigenschaften. $LTB_4$ ist das stärkste unter den bekannten Leukozyt-chemotaktischen Mitteln. $LTC_4$, $LTD_4$ und $LTE_4$ sind die Komponenten des "slow-reacting substance of anaphylaxis" (SRS-A) und induzieren Kontraktion der Bronchien.

Leukotriene werden in Zusammenhang mit der Pathogenese von verschiedenen vaskulären und pulmonalen Störungen unter Einbezug der Aktivierung von Leukozyten und glatter Muskulatur gebracht. Da diese Substanzen durch Biotransformation der Arachidonsäure (AA) mit 5-Lipoxygenase abgeleitet werden, unterdrückt selektive Hemmung von 5-Lipoxygenase damit ihre Bildung in Leukozyten und anderen Organsystemen.

5-Lipoxygenase-Hemmung wird z.B. durch die Messung der prozentualen Hemmung der Synthese von 5-HETE [(5S)-5-Hydroxy-6,8,11,14-eicosa-tetraensäure] und Leukotrien $B_4$ ($LTB_4$, 5,12-Di-hydroxy-6,8,10,14-eicosatetraensäure) in A-23187 - stimulierten polymorphonuklearen Leukozyten des Meerschweinchens bestimmt, vor allem nach der radiometrischen Dünnschichtchromatographie - beschrieben von Walker und Dawson (J.Pharm. Pharmacol. 31 778, 1979) und Jakschik und Lee (Nature 287: 51, 1980), gemessen wird die Bildung von 5-HETE und $LTB_4$-artigen Produkten aus der $^{14}C$-Arachidonsäure. $IC_{50}$-Werte werden graphisch als die Konzentration einer Test-Verbindung bestimmt, bei der die Synthese von 5-HETE und $LTB_4$-Produkten zu 50 % reduziert wird (im Hinblick auf die Kontrollwerte).

Die 5-Lipoxygenase-Hemmung in vivo wird nach oraler oder intravenöser Verabreichung an Ratten oder Hunden durch Bestimmung der Hemmung von A-23187-stimulierten $LTB_4$ Bildung im Blut (ex vivo) als Vergleich zu den nicht behandelten Kontrolltieren ausgewertet.

Die antiinflammatorischen Eigenschaften der erfindungsgemässen Verbindungen werden durch Bestimmung der Fähigkeit ermittelt, die Freisetzung der Leukozyten und der Oedembildung, welche durch Carrageein-Injektion an Ratten induziert wurden, zu verhindern (z.B. gemäss A.P. Almeida et al., J.Pharmacol.Exp.Therap.214, 74 (1980).

So ist z.B. das 4-(N-Methylcyclohexylamino)-1,2-diacetoxynaphthalin, als repräsentative Verbindung der Formel I der vorliegenden Erfindung, hochaktiv und hemmt die Bildung von 5-HETE [(5S)-5-Hydroxy-6,8,11,14-eicosatetraensäure] und Leukotrien $B_4$, ($LTB_4$, 5,12-Di-hydroxy-6,8,10,14-eicosatetraensäure) im A-23187-stimulierten polymorphonuklearen Leukozyten des Meerschweinchens, z.B. bei $IC_{50}$ von etwa einem mikromolar ($1 \times 10^{-6}$ M) für $LTB_4$-Bildung. Diese erfindungsgemässe Verbindung hemmt auch die 5-Lipoxygenaseaktivität (ex vivo bestimmt) bei einer Dosis von 1 mg/kg intravenös oder 100 mg/kg p.o. bei den Versuchen mit Hunden und bei einem Dosisbereich von etwa 10-100 mg/kg p.o. bei den Versuchen mit Ratten.

Bei Verabreichung von 4-(N-Methylcyclohexylamino)-1,2-diacetoxynaphthalin 30 mg/kg p.o während zwei Tagen (zweimal pro Tag) wird in einem inflammatorischen Modell bei der Ratte bewirkt, dass 48 Stunden nach einer Carraeein-Injektion die dadurch verursachte Bildung von polymorphonuklearen Leukozyten und Monozyten herabgesetzt wird.

Die besagten vorteilhaften Eigenschaften belegen die nützliche therapeutische Verwendbarkeit der erfindungsgemässen Verbindungen in Säugern (inkl. Menschen) als Arzneimittel zur Behandlung von

Krankheiten, welche durch 5-Lipoxygenase Wirkung oder Anhäufung von Leukozyten (z.B. Neutrophilen) verursacht werden, insbesondere allergischen und inflammatorischen Krankheiten, z.B. Lungenallergien und inflammatorischen Krankheiten, wie Asthma, dermatologischen Allergien und inflammatorischen Krankheiten der Haut, wie Psoriasis, arthritischen inflammatorischen Krankheiten, wie rheumatische Arthritis, und auch allergischen und inflammatorischen Krankheiten des Auges, oder von ischämischen Krankheiten, wie Myocardialinfarkt.

Die erfindungsgemässen Verbindungen können nach folgenden Verfahren hergestellt werden:

a) Reduktion einer Verbindung der Formel III

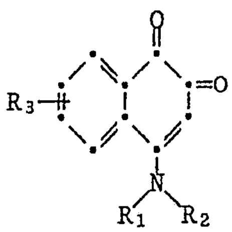

(III)

worin $R_1$, $R_2$ und $R_3$ die oben genannten Bedeutungen haben, mit einem geeigneten Reduktionsmittel und Umwandlung einer erhaltenen Verbindung der Formel I in ein Esterderivat davon oder in eine andere Verbindung der Erfindung; oder

b) zur Herstellung einer Verbindung der Formel I, worin $R_1$ und $R_2$ mit dem N-Atom keinen cyclischen Substituenten bilden, Reduktion einer Verbindung der Formel IV

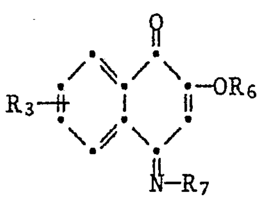

(IV)

worin $R_6$ Niederalkyl oder Aryl-niederalkyl bedeutet, $R_7$ für die Bedeutung von $R_1$ in der Definition von Verbindungen der Formel II steht, und $R_3$ die obengenannte Bedeutung hat, so dass man eine Verbindung der Formel V erhält,

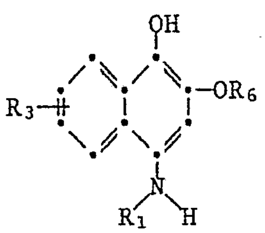

(V)

worin $R_1$, $R_3$ und $R_6$ die obengenannten Bedeutungen haben, und Umwandlung einer erhaltenen Verbindung der Formel V in eine andere Verbindung der Erfindung, z.B. eine solche, worin die freie Hydroxygruppe verestert ist und/oder worin $R_2$ eine andere Bedeutung als Wasserstoff hat; wobei man gewünschtenfalls reaktionsfähige funktionelle Gruppen intermediär bei der Ausführung eines der genannten Verfahren schützt und anschliessend die freie Verbindung isoliert, und/oder eine erhaltene Verbindung der Formel I in eine andere Verbindung der Formel I umwandelt, und/oder eine erhaltene freie Verbindung in ein Salz oder ein Salz in die freie Verbindung oder in ein anderes Salz umwandelt; und/oder ein erhaltenes Gemisch von Isomeren oder Racematen in die einzelnen Isomere oder Racemate auftrennt, und/oder ein Racemat in die optischen Antipoden aufspaltet.

Die in den Ausgangsmaterialien und Zwischenprodukten vorhandenen funktionellen Gruppen, wie Amino- und Hydroxy-Gruppen, sind gegebenenfalls durch solche Schutzgruppen (conventional protecting groups) geschützt, die üblicherweise in der präparativen Chemie verwendet werden. Die Amino- und Hydroxy-Schutzgruppen können unter schonenden Reaktionsbedingungen, das heisst unter Vermeidung von Spaltung sowie anderen Nebenreaktionen, abgespalten werden.

Die bekannten Schutzgruppen mit den gewünschten Eigenschaften sind beispielsweise in J.F.W. McOmie "Protective Groups in Organic Chemistry", Plenum Press, London, New York, 1973, T.W. Greene, "Protective Groups in Organic Synthesis", Wiley, New York, 1984, beschrieben.

Die Herstellung von erfindungsgemässen Verbindungen durch Reduktion gemäss der Verfahrensvariente a) kann nach bekannten Verfahren für Umwandlung von ortho-Chinon-Verbindungen in die entsprechenden vicinal aromatischen Diole durchgeführt werden. Die Reduktion wird vorzugsweise unter Verwendung von Hydrierung, mit Wasserstoff in Gegenwart von entsprechenden Katalysatoren, z.B. Palladium auf Aktivkohle, in einem Lösungsmittel, wie Dichlormethan, durchgeführt.

Die erfindungsgemässen Verbindungen werden vorzugsweise mit einer oder beiden Hydroxygruppen in geschützter Form isoliert. Die Hydrierung wird bevorzugt bei Raumtemperatur, unter atmosphärischem oder erhöhtem Druck durchgeführt. Die Reduktion von Verbindungen der Formel III wird vorzugsweise in Gegenwart von Acylierungsmitteln durchgeführt, damit man direkt eine erfindungsgemässe Verbindung der Formel I, worin eine oder beide Hydroxygruppen verestert sind. herstellt. Geeigneterweise werden dabei die Acylierungsmittel. z.B. Essigsäureanhydrid, als Lösungsmittel bei der Hydrierung verwendet.

Acylierungsmittel für die oben erwähnte Herstellung von Esterderivaten sind aus dem Stand der Technik bekannt.

Säureanhydride oder -halogenide sind z.B. geeignete Acylierungsmittel zur Herstellung von Niederalkancarbonsäure-, Arylcarbonsäure-. Aryl-niederalkancarbonsäure- und Niederalkoxy-niederalkancarbonsäureestern. Isocyanate sind geeignet zur Herstellung von Mono-(niederalkyl, Aryl oder Arylniederalkyl)-carbaminsäureestern; N,N'-Disubstituierte Carbamoylchloride sind geeignet zur Herstellung von Di(Niederalkyl, Aryl oder Arylniederalky)carbaminsäureestern; 1,1'-Carbonyldiimidazole sind geeignet zur Herstellung von Esterderivaten, worin beide Hydroxygruppen in Form von cyclischen Carbonatestern vorliegen.

Die Reduktion (a) wird insbesondere mit Tri-(niederalkyl)-phosphit, wie Triethylphosphit, durchgeführt, um direkt eine Di-(Niederalkyl)-phosphorsäureester-Verbindung der Formel I zu bekommen.

Die Ausgangsverbindung der Formel III können nach technisch bekannten Methoden durch Umsetzung von 1,2-Naphthochinon-Derivaten der Formel VI

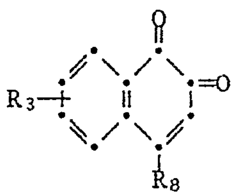

(VI)

worin $R_8$ eine Abgangsgruppe darstellt, mit einem Amin der Formel VII

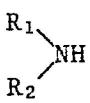

(VII)

worin $R_1$ und $R_2$ unabhängig voneinander oder zusammen die obengenannte Bedeutung haben, hergestellt werden.

Eine Abgangsgruppe $R_8$ ist z.B. Chlor, Brom oder Jod. und insbesondere die Sulfonsäuregruppe als Natrium- oder Kaliumsalz; $R_8$ kann auch Niederalkoxy, wie Ethoxy, bedeuten.

Die Herstellung von diesen Zwischenprodukten der Formel III wird vorzugsweise durch Umsetzung von einem Salz, insbesondere einem Alkalimetallsalz, wie Natriumsalz, von einer gegebenenfalls substituierten 1,2-Naphthochinon-4-sulfonsäure (eine Verbindung der Formel VI, worin $R_8$ $SO_3Na$ bedeutet) in einem geeigneten Lösungsmittel insbesondere Wasser, bei einem Temperaturbereich von etwa +5°C bis +90°C, insbesondere bei Raumtemperatur, mit einem Amin der Formel VII durchgeführt.

Die Amine der Formel VII sind entweder bekannt oder können nach an sich bekannten methoden hergestellt werden.

Auch die 1,2-Naphthochinonderivate der Formel VI sind entweder bekannt oder können nach an sich bekannten Methoden hergestellt werden.

Die Reduktion gemäss Verfahren (b) kann in gleicher Weise durchgeführt werden wie im Verfahren (a) beschrieben. In den erhaltenen Verbindungen der Formel V kann die freie Hydroxygruppe entweder wie oben beschrieben acyliert oder in an sich bekannter Weise alkyliert werden. In den erhaltenen Verbindungen der Formel V kann auch die sekundäre Aminogruppe nachträglich entweder N-acyliert werden, z.B. in Gegenwart von Zinkchlorid, um erfindungsgemässe Verbindungen zu erhalten, worin $R_2$ für Niederalkanoyl oder Arylniederalkanoyl steht; oder N-alkyliert werden, um erfindungsgemässe Verbindungen, worin $R_2$ Niederalkyl oder Arylniederalkyl bedeutet, zu bekommen.

Die N-Alkylierung wird nach an sich bekannten Methoden durchgeführt, z.B. durch Kondensation mit einem reaktionsfähigen Derivat eines entsprechenden Niederalkyl- oder Arylniederalkylalkohols, z.B. einem Halogenid, wie Bromid oder Jodid, gegebenenfalls in Gegenwart von einer Base, wie Triethylamin oder Pyridin, in einem Lösungsmittel, z.B. Acetonitril oder Dichlormethan, bei Raumtemperatur oder unter Erwärmen zum Siedepunkt des verwendeten Lösungsmittel.

Die Ausgangsverbindungen der Formel IV werden bevorzugt durch Umsetzung von einer Verbindung der Formel III, worin $R_2$ Wasserstoff bedeutet (d.h. dem 2-Hydroxy-4-iminotautomer davon), in einer Lauge, z.B. 30 %iger wässriger Natriumhydroxidlösung, z.B. mit einem reaktionsfähigen Niederalkanolderivat, wie Diniederalkylsulfat, insbesondere Dimethylsulfat, hergestellt.

Die Dihydroxy substituierten Verbindungen der Formel I können, insbesondere in situ, in an sich bekannter Weise in andere erfindungsgemässe Verbindungen umgewandelt werden. Beispielsweise kann eine Verbindung der Formel I in ein Esterderivat oder Diesterderivat davon wie unter dem Verfahren (a) beschrieben umgewandelt werden. Eine Verbindung der Formel I kann auch in ein Etherderivat, insbesondere in ein Dietherderivat, in an sich bekannter Weise, z.B. in Gegenwart von einer Base, wie wässriger Natriumhydroxidlösung, in inerter Atmosphäre mit geeigneten Veretherungsmitteln, insbesondere einem reaktionsfähigen funktionellen Derivat des entsprechenden verethernden Alkohols, wie Diniederalkylsulfat oder Niederalkylhalogenid, umgewandelt werden.

Ein Esterderivat einer erfindungsgemässen Verbindung der Formel I kann in eine Verbindung der Formel I, mit freien Hydroxygruppen z.B. mittels verdünnter Mineralsäure, wie verdünnte Salzsäure, bei Raumtemperatur unter Inertatmosphäre, umgewandelt werden.

Eine erfindungsgemässe Verbindung der Formel I, worin $R_1$ und $R_2$ Wasserstoff bedeuten, kann zu einer entsprechenden Verbindung, worin das Stickstoffatom durch Niederalkanoyl oder Arylniederalkanoyl acyliert ist, durch Behandlung z.B. mit dem entsprechenden Säureanhydrid, insbesondere in Gegenwart einer geeigneten Lewissäure, wie wasserfreiem Zinkchlorid, umgewandelt werden.

Die oben genannten Reaktionen werden nach Standverfahren durchgeführt, in Gegenwart oder Abwesenheit von Verdünnungsmitteln, vorzugsweise solchen, welche gegenüber den Reagenzien inert sind und diese lösen, von Katalysatoren, Kondensations- oder anderen oben genannten Mitteln, und/oder in einer inerten Atmosphäre, unter Kühlung, bei Zimmertemperatur oder bei erhöhter Temperatur, vorzugsweise beim Siedepunkt des verwendeten Lösungsmittels, und bei normalem oder erhöhtem Druck.

Die bevorzugten Lösungsmittel, Katalysatoren und Reaktionsbedingungen werden in den Ausführungsbeispielen angegeben.

Die Erfindung betrifft ebenfalls Abänderungen der vorliegenden Verfahren, wonach ein auf irgendeiner Stufe des Verfahren erhaltenes Zwischenprodukt als Ausgangsmaterial verwendet wird und die verbleibenden Verfahrensschritte durchgeführt werden, oder das Verfahren auf irgendeiner Stufe abgebrochen wird, oder wonach ein Ausgangsmaterial unter den Reaktionsbedingungen gebildet, oder worin ein Ausgangsstoff in Form eines Salzes oder optisch reinen Antipoden verwendet wird. Immer wenn gewünscht, werden die oben genannten Verfahren erst durchgeführt, nachdem alle potentiell störenden, reaktionsfähigen funktionellen Gruppen in geeigneter Weise geschützt sind, z.B. wie oben und in den Beispielen illustriert.

In den genannten Reaktionen werden vorteilhafterweise solche Ausgangsstoffe verwendet, welche zu den weiter vorn als besonders bevorzugt beschriebenen Verbindungen führen.

Die Erfindung betrifft auch neue Ausgangsstoffe und Verfahren zu ihrer Herstellung.

Abhängig von den gewählten Ausgangsstoffen und Methoden können die neuen Verbindungen in Form von Isomeren oder als Isomerengemische vorliegen, zum Beispiel als geometrische Isomeren oder Mischungen davon (cis oder trans), als optisch reine Antipoden, Racemate oder Diastereomeren. Die obengenannten Isomere oder Isomergemische gehören auch in den Bereich der vorliegenden Erfindung.

Erhaltene geometrische oder diastereomere Isomerengemisch der obigen Verbindungen oder Zwischenprodukte können nach an sich bekannten Methoden in die einzelnen racemischen oder optisch aktiven Isomeren getrennt werden, z.B. durch fraktionierte Destillation, Kristallisation und/oder Chromatographie. Racemische Produkte können gleichfalls in die optischen Antipoden gespalten werden, z.B. durch Trennung ihrer diastereomeren Salze, wie gemäss J. Org. Chem. 43, 3803 (1978), z.B. durch fraktionierte Kristallisation von d- und ℓ-(Tartraten, Mandelaten, Camphersulfaten oder Dibenzoyltartraten) Salzen.

Bevorzugt wird jeweils das wirksamere Isomere und/oder der wirksamere Antipode der erfindungsgemässen Verbindungen isoliert.

Die Verbindungen der vorliegenden Erfindung werden entweder in freier oder in Form ihrer Salze erhalten. Jede erhaltene Base kann in das entsprechende Säureadditionssalz umgewandelt werden, vorzugsweise unter Verwendung einer therapeutisch annehmbaren Säure oder eines Anionenaustauschers. Erhaltene Salze können in die entsprechenden freien Basen umgewandelt werden, zum Beispiel unter Verwendung einer stärkeren Base, beispielsweise eines Metal- oder Ammoniumhydroxids oder eines basischen Salzes, z.B. eines Alkalimetallhydroxids oder -carbonats, oder eines Kationenaustauschers. Diese oder andere Salze, etwa Pikrate, können auch zur Reinigung der erhaltenen Basen verwendet werden, indem man die Basen in Salze überführt, diese abtrennt und reinigt, und aus den Salzen wiederum die Base freisetzt. Infolge der engen Beziehung zwischen den freien Verbindungen und ihren Salzen sind in diesem Zusammenhang unter freien Verbindungen sinn- und zweckmässig gegebenenfalls immer auch die entsprechenden Salze zu verstehen.

Die Verbindungen und ihrer Salze können auch in form ihrer Hydrate erhalten werden, oder sie können andere zur Kristallisation verwendete Lösungsmittel einschliessen.

Die Zwischenprodukte der Formel III und pharmazeutisch annehmbare Salze davon zeigen auch selektive Lipoxygenase hemmende Eigenschaften und sind in gleicher Weise wie die erfindungsgemässen Verbindungen der Formel I pharmakologisch verwendbar. Die vorliegende Erfindung betrifft daher ebenfalls die Verbindungen der Formel III und insbesondere ihre Verwendung als Lipoxygenase-Hemmer. Insbesondere Verbindungen der Formel III, worin $R_1$ $C_5$-$C_7$-Cycloalkyl, Bicyclo[2.2.1]heptyl, Admantyl, 1- oder 2-Indanyl oder Perhydroindanyl, oder 1- oder 2-Tetrahydro- oder Perhydro- naphthyl bedeutet, $R_2$ Wasserstoff, Niederalkyl, Arylniederalkyl, $C_5$-$C_7$-Cycloalkyl-niederalkyl, Niederalkanoyl oder Arylniederalkanoyl bedeutet; oder $R_1$ und $R_2$ zusammen mit dem Stickstoffatom, an welches sie gebunden sind, für Pyrrolidino, Piperidino, Perhydroazepino, Morpholino, Thimorpholino, Piperazino oder N-Niederalkyl-piperazino, Tetrahydro- oder Perhydro-isochinolinyl, Tetrahydro- oder Perhydrochinolinyl, Dihydro- oder Perhydroindolyl stehen; $R_3$ Wasserstoff, Niederalkyl oder Halogen bedeutet; und pharmazeutisch annehmbare Säureadditionssalze davon.

Besonders bevorzugt sind Verbindungen der Formel III, worin $R_1$ Cyclopentyl, Cyclohexyl, Cycloheptyl, 2-Norbornyl, 1- oder 2-Admantyl, 1- oder 2-Indanyl, oder 1- oder 2-Perhydroindanyl bedeutet; $R_2$ Wasserstoff, $C_1$-$C_4$-Alkyl oder Aryl-$C_1$-$C_4$-alkyl bedeutet; und $R_3$ Wasserstoff ist, und pharmazeutisch annehmbare Säureadditionssalze davon.

Bevorzugt sind dann Verbindungen der Formel III, worin $R_1$ und $R_2$ zusammen mit dem Stickstoffatom, an welches sie gebunden sind, für Tetrahydro- oder Perhydro-isochinolinyl, Tetrahydro- oder Perhydrochinolinyl, Dihydro-oder Perhydroindolyl stehen; und $R_3$ Wasserstoff bedeutet, und pharmazeutisch annehmbare Säureadditionssalze davon.

So hemmt z.B. das 4-(N-Methyl-cyclohexylamino)-1,2-naphthochinon, als repräsentative Verbindung der Formel III, die Bildung von $LTB_4$ in A-23187-stimulierten polymorphonuklearen Leukozyten des Meerschweinchens bei $IC_{50}$ von etwa 0,5 mikromolar in vitro.

Die genannte 4-(N-Methylcyclohexylamino)-1,2-napthochinon-Verbindung wird auch in vivo nach Verabreichung als Metabolit von 4-(Methylcyclohexylamino)-1,2-diacetoxynapthalin identifiziert.

Die vorliegende Erfindung betrifft ebenfalls die Verwendung von erfindungsgemässen Verbindungen zur Herstellung von pharmazeutischen Zusammensetzungen mit 5-Lipoxygenase hemmender Wirkung, welche eine wirksame Menge einer erfindungsgemässen Verbindung allein oder in Kombination mit einem oder mehreren pharmazeutisch annehmbaren Trägermaterialien enthalten.

Die pharmakologisch aktiven Verbindungen der Erfindung sind bei der Herstellung von pharmazeutischen Zusammensetzungen verwendbar, die eine wirksame Menge derselben in Verbindung oder Beimischung mit Excipienten oder Trägern, die für die enterale, transdermale oder parenterale Anwendung geeignet sind, umfassen. Bevorzugt sind Tabletten und Gelatinekapseln, die den aktiven Bestandteil zusammen mit a) Verdünnungsmitteln, z.B. Lactose, Dextrose, Sucrose, Mannit, Sorbit, Cellulose und/oder Glycin, b) Gleitmitteln, z.B. Siliciumdioxid, Talk, Stearinsäure, deren Magnesium- oder Calciumsalz und/oder Polyethylenglykol, für Tabletten auch c) Bindemitteln, z.B. Magnesiumaluminiumsilikat, Stärkepaste, Gelatine, Tragant, Methylcellulose, Natriumcarboxymethylcellulose und/oder Polyvinylpyrrolidon, gewünschtenfalls d) Zerteilungs- bzw. Desintegrationsmitteln, z.B. Stärken, Agar, Alginsäure oder deren Natriumsalz, oder schäumenden Mischungen und/oder e) Absorbentien, farbgebenden Mitteln, Geschmacksstoffen und süssenden Mitteln umfassen. Injizierbare präparate sind vorzugsweise wässrige isotonische Lösungen oder Suspensionen, und Suppositorien werden vorteilhaft aus Fettemulsionen oder -suspensionen hergestellt. Diese Zusammensetzungen können sterilisiert werden und/oder Ajuvanzien, wie Konservierungs-, Stabilisierung-, Netz- oder Emulgiermittel, Lösungspromotoren, Salze für die Regulierung des osmotischen Drucks und/oder Puffer, enthalten. Zusätzlich können sie auch andere therapeutisch wertvolle Substanzen enthalten. Diese Präparate werden nach herkömmlichen Misch-, Granulier- bzw. Ueberzugsmethoden hergestellt und enthalten etwa 0,1 bis 75 %, vorzugsweise etwa 1 bis 50 %, des aktiven Bestandteils.

Geeignete Formulierungen für die transdermale Anwendung enthalten eine wirksame Menge einer erfindungsgemässen Verbindung zusammen mit Trägermaterial. Geeignete Trägermaterialien umfassen pharmazeutisch annehmbare Hilfsstoffe, welche den Durchgang durch die Haut ermöglichen. Insbesondere haben transdermale therapeutische Systeme die Form eines Pflasters mit einer Schutzschicht, einem Wirkstoffreservoir, gegebenenfalls mit Trägermaterialien, und einer die Abgabe bestimmenden Kontrollschicht, durch welche der Wirkstoff auf die Haut mit kontrollierter und bestimmbarer Geschwindigkeit über einen längeren Zeitabschnitt abgegeben wird. Das System hat gegebenenfalls noch eine Klebschicht.

Die vorliegende Erfindung betrifft ebenfalls die Verwendung von erfindungsgemässen Verbindungen mit 5-Lipoxygenase hemmenden Eigenschaften und pharmazeutischen Zusammensetzungen, die diese Verbindungen enthalten, für doe Behandlung von Erkrankungen bei Säugern inkl. Menschen, deren Erkrankungen durch 5-Lipoxygenase-Hemmer regulierbar sind, insbesondere inflammatorische und allergische Krankheiten, unter Verwendung einer wirksamen Menge einer erfindungsgemässen Verbindung, vorzugsweise in Form der oben genannten pharmazeutischen Zusammensetztungen.

Die applizierbare Wirkstoffdosis ist speziesabhängig sowie vom Körpergewicht, dem Alter und individuellen Zustand und der Darreichungsform abhängig.

Die Einheitsdosis für einen Säuger, z.B. Mensch, von ca. 50 bis 70 kg Gewicht beträgt zwischen 10 und 200 mg des Wirkstoffs.

Die folgenden Beispiele sollen die Erfindung erläutern und keine Einschränkungen darstellen. Die Temperaturen sind in Celsiusgraden angegeben, und sämtliche Teile sind in Form von Gewichtsteilen

angegeben. Wenn nicht anders erwähnt, werden sämtliche Verdampfungen unter vermindertem Druck vorzugsweise zwischen etwa 15 und 100 mg Hg durchgeführt.

Die Stuktur der Endprodukte, Zwischenprodukte und Ausgangsmaterialien wurde mittels analytischer Methoden bestätigt, z.B. Mikroanalyse und spektroskopische Untersuchungen (z.B. MS, IR, NMR).

Beispiel 1

Eine Suspension von 3,5 g 4-(N-Methylcyclohexylamino)-1,2-naphthochinon in 50 ml Essigsäureanhydrid wird beim Druck von 3,1 bar und bei Raumtemperatur unter Verwendung von 1,2 g 10 % Palladium-auf-Aktivkohle als Katalysator hydriert. Nachdem die Aufnahme von Wasserstoff aufhört und die rote Verfärbung der Lösung verschwindet (5 Stunden), wird der Katalysator durch Filtration entfernt. Das Essigsäureanhydrid wird im Vakuum bei 60°C abgedampft und der Rückstand wird in Toluol aufgenommen und mit Aktivkohle behandelt und abfiltiert. Die Toluollösung wird im Vakuum abgedampft und das Produkt aus 1:1 Hesan-Diethylether-Gemisch (100 ml) kristallisiert, man erhält 4-(N-methylcyclohexylamino)-1,2-diacetoxy naphthalin, Verbindung der Formel II, worin $NR_1R_2$ N-Methylcyclohexylamino, $R_3$ Wasserstoff, und $R_4$ und $R_5$ Acetyl bedeuten, Smp. 103-105°C, Hydrochloridsalz Smp. 165-167°C.

Das Ausgangsmaterial wird folgendermassen erhalten:

Eine Lösung von 10,4 g 1,2-Naphthochinon-4-sulfonsäure-natriumsalz in 350 ml Wasser wird bei Raumtemperatur während 30 Minuten gerührt. Zu dieser Lösung wird in einer Portion 4,25 g N-Methylcyclohexylamin zugefügt. Die Lösung wird 5 Stunden bei Raumtemperatur gerührt und der Rückstand abfiltriert. Das Produkt wird mit 200 ml Wasser gewaschen und bei 100°C im Vakuum über Nacht getrocknet, man erhält 4-(N-Methylcyclohexylamino)-1,2-napthochinon mit einem Schmelzpunkt von 168-170°C. Die Herstellung von der gleichen Verbindung aus 4-Ethyloxy-1,2-naphthochinon ist in J.Med.Chem. 13, 97 (1970) beschrieben.

Beispiel 2

In analoger Weise wie im Beispiel 1 beschrieben werden folgende Verbindungen der Formel II, worin $R_4$ und $R_5$ Acetyl, $R_3$ Wasserstoff und $NR_1R_2$ die unten angegebene Bedeutung haben, hergestellt:

| $NR_1R_2$ | Schmelzpunkt (°C) |
|---|---|
| a) N-Ethylcyclohexylamino | 95 - 97° |
| b) Tetrahydroisochinolinyl | 133 - 135° |
| c) Perhydroisochinolinyl | 114 - 116° |
| d) Perhydrochinolinyl | 143 - 145° |
| e) N-Methylcycloheptylamino | 93 - 95° |
| f) N-Methyl-exo-2-norbornylamino | 105 - 107° |
| g) N-Methyl-1-indanylamino | 142 - 144° |
| h) 1-Adamantylamino | 84 - 86° |
| i) 2-Adamantylamino | 117 - 120° |
| j) Tetrahydrochinolinyl | --- |
| k) N-(1-Methylpiperidin-4-yl)-methylamino | --- |
| l) Cyclohexylamino | --- |

Die Ausgangsverbindungen sind die entsprechenden 1,2-Naphthochinonverbindungen der Formel III, worin $R_3$ Wasserstoff und $NR_1R_2$ die unten definierte Bedeutung haben.

| $NR_1R_2$ | Schmelzpunkt (°C) |
|---|---|
| a) N-Ethylcyclohexylamino | --- |
| b) Tetrahydroisochinolinyl | 256 - 258° |
| c) Perhydroisochinolinyl | 251 - 253° |
| d) Perhydrochinolinyl | 223 - 225° |
| e) N-Methylcycloheptylamino | 260° |
| f) N-Methyl-exo-2-norbornylamino | 138 - 140° |
| g) N-Methyl-1-indanylamino | 258 - 260° |
| h) 1-Adamantylamino | grösser 180° |
| i) 2-Adamantylamino | --- |
| j) Tetrahydrochinolinyl | grösser 250° |
| k) N-(1-Methylpiperidin-4-yl)-methylamino | 183 - 185° |
| l) Cyclohexylamino | 220 - 222° |

Beispiel 3

a) Eine Lösung von 3,0 g 4-(N-Methylcyclohexylamino)-1,2-napthochinon und 4,8 g 1,1'-Carbonyldiimidazol in 150 ml Dichlormethan wird bei Druck von 3,1 bar und bei Raumtemperatur unter Verwendung von 1,5 g 10 % Palladium-auf-Aktivkohle als Katalysator hydriert. Nachdem die Aufnahme von Wasserstoff aufhört und die rote Verfärbung der Lösung verschwindet (5 Stunden), wird der Katalysator durch Filtration entfernt. Das Dichlormethan wird im Vakuum abgedampft und der Rückstand in 250 ml Diethylether und 150 ml Wasser 16 Stunden gerührt. Die organische Phase wird abgetrennt und mit 100 ml Kochsalzlösung gewaschen und dann über Magnesiumsulfat getrocknet und filtriert. Der Diethylether wird im Vakuum entfernt und der Rückstand in 200 ml Dichlormethan aufgenommen und durch 100 g Silicagel filtriert. Das Produkt wird aus 50 ml Diethylether kristallisiert, und man erhält 4-(N-Methylcyclohexylamino)-1,2-carbonyldioxynaphthalin, Schmelzpunkt 132-134° C.

b) In analoger Weise wird aus 4-(N-Methylcyclohexylamino)-1,2-naphthochinon bei Verwendung von 5 Mol p-Chlorphenylisocyanat als Acylierungsmittel 4-(N-Methylcyclohexylamino)-1-(N-p-chlorphenylcarbamoyloxy)-2-hydroxy-naphthalin hergestellt, Schmelzpunkt 108-110° C.

c) In analoger Weise wie im Beispiel (b) wird aus 4-(N-Methylcyclohexylamino)-1,2-naphthocyinon durch Hydrierung des Reaktionsgemisches bei Raumtemperatur während 20 Stunden 4-(N-Methylcyclohexylamino)-1,2-di-(N-p-chlorhexylcarbamoyloxy)-napthalin, Schmelzpunkt 173-175° C, erhalten.

Beispiel 4

Eine Lösung von 3,0 g 4-(N-Methylyclohexylamino)-1,2-naphthocyinon in 150 ml Dichlormethan wird bei Raumtemperatur 20 Minuten gerührt. Zu dieser Lösung wird ein Ueberschuss von Triethylphosphit (8,5 ml) zugefügt. Die Lösung wird während 6 Stunden gerührt und dann 150 ml Wasser zugefügt. Das Zweiphasengemisch wird kräftig während 16 Stunden bei Raumtemperatur gerührt.

Die organische Phase wird abgetrennt, mit 100 ml Kochsalzlösung gewaschen, über Magnesiumsulfat getrocknet und abfiltriert. Das Dichlormethan wird im Vakuum abgedampft und der Rückstand in Hexan aufgenommen, mit Aktivkohle behandelt und filtriert. Das erhaltene 4-(N-Methylcyclohexylamino)-1-hydroxy-2-(diethylphosphonyloxy)-naphthalin wird aus 100 ml Hexan kristallisiert, Schmelzpunkt 134-136° C.

Beispiel 5

Eine Suspension von 4-Cyclohexylimino-1-oxo-2-methoxy-1,4-dihydronaphthalin in Essigsäureanhydrid wird wie im Beispiel 1 beschrieben hydriert, und man erhält 4-Cyclohexylamino-1-acetoxy-2-methoxynapthalin mit Schmelzpunkt 134-136° C.

Das Ausgangsmaterial wird folgendermassen hergestellt:

Eine Lösung von 9,0 g 4-(Cyclohexylamino)-1,2-naphthochinon in 200 ml Dimethylsulfat und 460 ml 30 %iger wässriger Natriumhydroxidlösung wird langsam zum Rückfluss erhitzt. Das Reaktionsgemisch wird während 6 Stun den gerührt, mit 300 ml Wasser gewaschen und bei 80°C im Vakuum getrocknet, und man erhält 4-Cyclohexylimino-1-oxo-2-methoxy-1,4-dihydronaphthalin, Schmelzpunkt 145-147°C.

Beispiel 6

Eine Lösung von 5,0 g 4-(Cyclohexylamino)-1-acetoxy-2-methoxynaphthalin in 75 ml Essigsäureanhydrid wird mit 500 mg Zinkchlorid bei 160-170°C während 2 Stunden gerührt. Das Reaktionsgemisch wird dann abgekühlt, filtriert und unter vermindertem Druck abgedampft. Der Rückstand wird in 300 ml Diethylether aufgenommen und mit 100 ml Wasser und dann mit 100 ml Kochsalzlösung gewaschen. Die organische Phase wird über Magnesiumsulfat getrocknet, filtriert und zur Trockenheit eingedampft. Der Rest wird aus 120 ml 2:1 Diethylether/Hexan kristallisiert, man erhält 4-(N-Acetylcyclohexylamino)-1-acetoxy-2-methoxynaphthalin, Schmelzpunkt 137-139°C.

Beispiel 7

a) Herstellung von 10'000 Tabletten mit je 10 mg Wirkstoff:

Bestandteile:
4-(N-Methylcyclohexylamino)-1,2-diacetoxynaphthalin-hydrochlorid 100,00 g
Lactose 2.400,00 g
modifizierte Stärke 125,00 g
Polyethylenglycol 6.000 150,00 g
Magnesiumstearat 40,00 g
gereinigtes Wasser g.h

Herstellung:

Alle pulverförmigen Bestandteile werden durch ein Sieb mit Oeffnungen von 0,6 mm gegeben. Dann werden die Wirkstoffsubstanz, Lactose, Magnesiumstearat und die Hälfte der Stärke in einem geeigneten Mischer vermischt. Die andere Hälfte der Stärke wird in 65 ml Wasser suspendiert und die Suspension zu der siedenden Lösung des Polyethylenglycols in 260 ml Wasser hinzugesetzt. Die gebildete Paste wird zu den Pulvern gegeben, die granuliert werden, erforderlichenfalls mit einer zusätzlichen Menge Wasser. Die Granulate werden über Nacht bei 35°C getrocknet, durch ein Sieb mit Oeffnungen von 1,2 mm getrieben und zu Tabletten gepresst, wobei man mit konkaven, oben zweigeteilten Formen arbeitet.

b) Herstellung von 1000 Kapseln mit jeweils 10 mg an wirksamem Bestandteil.

Bestandteile:
4-(N-Methylcyclohexylamino)-1,2-diacetoxynaphthalin-hydrochlorid 10,00 g
Lactose 207,00 g
modifizierte Stärke 80,00 g
Magnesiumstearat 3,00 g

Herstellung:

Alle pulverförmigen Bestandteile werden durch ein Sieb mit Oeffnungen von 0,6 mm gedrückt. Dann wird die Wirkstoffsubstanz in einen geeigneten Mischer gegeben und zuerst mit dem Magnesiumstearat, dann mit Lactose und der Stärke bis zur Homogenität vermischt. Hartgelatinekapseln Nr. 2 werden jeweils mit 300 mg dieses Gemisches gefüllt, wobei man mit einer Kapselfüllmaschine arbeitet.

**Patentansprüche**

1. Verbindungen der Formel I

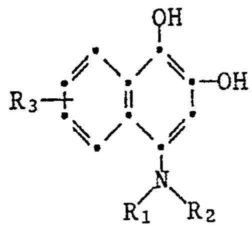

(I)

worin $R_1$ unsubstituiertes oder durch Niederalkyl substituiertes $C_3$-$C_7$-Cycloalkyl, unsubstituiertes oder durch Niederalkyl substituiertes $C_7$-oder $C_8$-Bicycloalkyl, unsubstituiertes oder durch Niederalkyl substituiertes Adamantyl. 4-Piperidinyl oder N-Niederalkyl oder Aryl-niederalkyl substituiertes Piperidinyl, 1- oder 2-Indanyl, 1- oder 2-Tetrahydronaphthyl, 1- oder 2-Perhydroindanyl, 1- oder 2-Perhydronapthyl bedeutet; $R_2$ Wasserstoff, Niederalkyl, $C_3$-$C_7$-Cycloalkyl, Aryl-niederalkyl, $C_3$-$C_7$-Cycloalkyl-niederalkyl, Niederalkanoyl oder Aryl-niederalkanoyl darstellt; oder $R_1$ und $R_2$, zusammen mit dem Stickstoffatom, an das sie gebunden sind, für Pyrrolidino, Piperidino, Perhydroazepino, Morpholino, Thiomorpholino, Tetrahydro- oder Perhydro-(isochinolinyl oder chinolinyl), Dihydro- oder Perhydro-indolyl, Piperazino oder N-Niederalkyl-piperazino stehen; $R_3$ Wasserstoff, Niederalkyl, Halogen oder Niederalkoxy bedeutet; pharmazeutisch annehmbare Ester- und Etherderivate davon; und pharmazeutisch verwendbare Säureadditionssalze der basischen Verbindungen der Formel I und ihrer Derivate.

2. Verbindungen gemäss Anspruch der Formel II

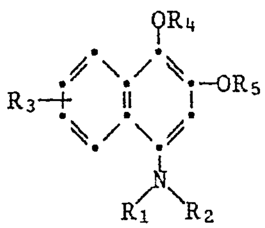

(II)

worin $R_1$ $C_5$-$C_7$-Cycloalkyl, Bicyclo[2.2.1]heptyl, Admantyl, 1- oder 2-Indanyl oder Perhydro-indanyl, oder 1- oder 2-Tetrahydro- oder Perhydro-naphthyl bedeutet, $R_2$ Wasserstoff, Niederalkyl, Arylniederalkyl, $C_5$-$C_7$-Cycloalkylniederalkyl, Niederalkanoyl oder Arylniederalkanoyl darstellt, $R_3$ Wasserstoff, Niederalkyl oder Halogen ist, $R_4$ und $R_5$ unabhängig voneinander Wasserstoff, Niederalkanoyl, Aroyl, Niederalkyl, N-Arylcarbamoyl, N-Mono- oder N,N-Di-Alkylcarbamoyl, oder O,O-Di-niederalkylphosphonyl, oder $R_4$ und $R_5$ zusammen Carbonyl bedeuten; und pharmazeutisch annehmbare Säureadditionssalze davon, vorausgesetzt, dass $R_2$ Wasserstoff, Niederalkyl, Arylniederalkyl oder $C_5$-$C_7$-Cycloalkylniederalkyl darstellt.

2. Verbindungen gemäss Anspruch 2 der Formel II, worin $R_1$ Cyclopentyl, Cyclohexyl, Cycloheptyl, 2-Norbornyl, 1- oder 2-Adamantyl, 1- oder 2-Indanyl oder 1- oder 2-Perhydro-indanyl bedeutet, $R_2$ Wasserstoff, $C_1$-$C_4$-Alkyl oder Aryl-$C_1$-$C_4$-alkyl ist, $R_3$ Wasserstoff, $C_1$-$C_4$-Alkyl oder Halogen bedeutet, $R_4$ Wasserstoff, $C_2$-$C_4$-Alkanoyl, N-Arylcarbamoyl, N-Mono- oder N,N-Di-$C_1$-$C_4$-Alkylcarbamoyl, und $R_5$ Wasserstoff, $C_1$-$C_4$-Alkyl, $C_2$-$C_4$-Alkanoyl, N-Arylcarbamoyl, N-Mono- oder N,N-Di-$C_1$-$C_4$-Alkylcarbamoyl oder O,O-Di-$C_1$-$C_4$-Alkylphosphonyl bedeuten; und pharmazeutisch annehmbare Säureadditionssalze davon.

4. Verbindungen gemäss Anspruch 2 der Formel II, worin $R_1$ Cyclopentyl, Cyclohexyl oder Cycloheptyl, $R_2$ Niederalkyl, $R_3$ Wasserstoff, $R_4$ $C_2$-$C_4$-Alkanoyl, und $R_5$ $C_2$-$C_4$-Alkanoyl bedeuten; und pharmazeutisch annehmbare Säureadditionssalze davon.

5. Verbindungen gemäss Anspruch 2 der Formel II, worin $R_1$ Cyclohexyl, $R_2$ $C_1$-$C_4$-Alkyl, $R_3$ Wasserstoff, $R_4$ und $R_5$ $C_2$-$C_4$-Alkanoyl bedeuten; und pharmazeutisch annehmbare Säureadditionssalze davon.

6. Verbindungen gemäss Anspruch 1 der Formel II

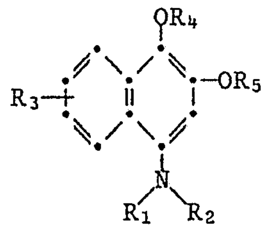

(II)

worin $R_1$ und $R_2$ zusammen mit dem Stickstoffatom, an welches sie gebunden sind, für Pyrrolidino, Piperidino, Perhydroazepino, Morpholino, Thiomorpholino, Piperazino oder N-Niederalkyl-piperazino, Tetrahydro- oder Perhydro-isochinolinyl, Tetrahydro-oder Perhydro-chinolinyl, Dihydro- oder Perhydro-indolinyl stehen, $R_3$ Wasserstoff, Niederalkyl oder Halogen ist, $R_4$ und $R_5$ unabhängig voneinander Wasserstoff, Niederalkanoyl, Aroyl, Niederalkyl, N-Arylcarbamoyl, N-Mono- oder N,N-Dialkylcarbamoyl oder O,O-Di-Niederalkylphosphonyl, oder $R_4$ und $R_5$ zusammen Carbonyl darstellen; und pharmazeutisch annehmbare Säureadditionssalze davon.

7. Verbindungen gemäss Anspruch 6 der Formel II, worin $R_1$ und $R_2$ zusammen mit dem Stickstoffatom, an welches sie gebunden sind, für Tetrahydro- oder Perhydro-isochinolinyl, Tetrahydro- oder Perhydro-chinolinyl, Dihydro- oder Perhydro-indolyl stehen, $R_3$ Wasserstoff, $R_4$ Wasserstoff, $C_2$-$C_4$-Alkanoyl, N-Arylcarbamoyl, N-Mono- oder N,N-Di-$C_1$-$C_4$-Alkylcarbamoyl, und $R_5$ Wasserstoff, $C_1$-$C_4$-Alkyl, $C_2$-$C_4$-Alkanoyl, N-Arylcarbamoyl, N-Mono- oder N,N-Di-Alkylcarbamoyl, oder O,O-Di-$C_1$-$C_4$-Alkylphosphonyl bedeuten; und pharmazeutisch annehmbare Säureadditionssalze davon.

8. Verbindungen gemäss Anspruch 6 der Formel II, worin $R_1$ und $R_2$ zusammen mit dem Stickstoffatom, an welches sie gebunden sind, für Tetrahydro- oder Perhydro-chinolinyl, Tetrahydroisochinolinyl oder Perhydroisochinolinyl stehen, $R_3$ Wasserstoff ist, $R_4$ und $R_5$ $C_2$-$C_4$-Alkanoyl bedeuten; und pharmazeutisch annehmbare Säureadditionssalze davon.

9. Verbindung gemäss Anspruch 3 der Formel II, worin $NR_1R_2$ für N-Methylcyclohexylamino und $R_3$ für Wasserstoff steht und $R_4$ und $R_5$ jeweils Acetyl bedeuten, bei der es sich um 4-(N-Methylcyclohexylamino)-1,2-diacetoxynapthalin handelt, und pharmazeutisch annehmbare Säureadditionssalze davon.

10. Verbindung gemäss Anspruch 3 der Formel II, worin $NR_1R_2$ für N-Methylexo-2-norbornyl-amino und $R_3$ für Wasserstoff steht und $R_4$ und $R_5$ jeweils Acetyl bedeuten, und pharmazeutisch annehmbare Säureadditionssalze davon.

11. Verbindung gemäss Anspruch 3 der Formel II, worin $NR_1R_2$ für Perhydroisochinolinyl und $R_3$ für Wasserstoff steht und $R_4$ und $R_5$ jeweils Acetyl bedeuten, und pharmazeutisch annehmbare Säureadditionssalze davon.

12. Pharmazeutische Präparate enthaltend Verbindungen der Ansprüche 1 bis 11 oder ein pharmazeutisch annehmbares Salz davon zusammen mit einem pharmazeutisch geeigneten Trägermaterial.

13. Die in den Ansprüchen 1 bis 11 genannten Verbindungen zur Anwendung in einem Verfahren zur therapeutischen Behandlung des menschlichen oder tierischen Körpers.

14. Die in den Ansprüchen 1 bis 11 genannten Verbindungen als 5-Lipoxygenase-hemmer.

15. Die in Anspruch 9 genannten Verbindungen als 5-Lipoxygenase-Hemmer.

16. Verwendung der in den Ansprüchen 1 bis 11 genannten Verbindungen zur Herstellung von pharmazeutischen Präparaten.

17. Verwendung der in den Ansprüchen 1 bis 11 genannten Verbindungen zur Herstellung von pharmazeutischen Präparaten zur Verwendung als 5-Lipoxygenase-Hemmer.

18. Verfahren zur Herstellung von den in Anspruch 1 genannten Verbindungen der Formel I, in der alle Symbole die in Anspruch 1 angegebenen Bedeutungen haben, und deren Salzen, gekennzeichnet durch

a) Reduktion einer Verbindung der Formel III

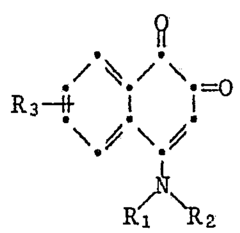

(III)

worin $R_1$, $R_2$ und $R_3$ die oben genannten Bedeutungen haben, mit einem geeigneten Reduktionsmittel und Umwandlung einer erhaltenen Verbindung der Formel I in ein Esterderivat

davon oder in eine andere Verbindung der Erfindung; oder

b) zur Herstellung einer Verbindung der Formel I, worin $R_1$ und $R_2$ mit dem N-Atom keinen cyclischen Substituenten bilden, Reduktion einer Verbindung der Formel IV

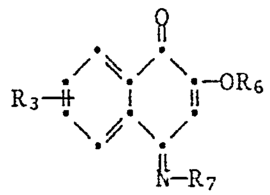

(IV)

worin $R_6$ Niederalkyl oder Aryl-niederalkyl bedeutet, $R_7$ für die Bedeutung von $R_1$ in der Definition von Verbindungen der Formel II steht, und $R_3$ die obengenannte Bedeutung hat, so dass man eine Verbindung der Formel V erhält,

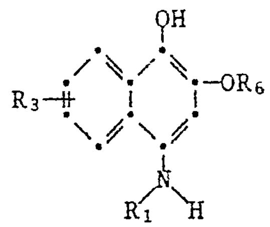

(V)

worin $R_1$, $R_3$ und $R_6$ die obengenannten Bedeutungen haben, und Umwandlung einer erhaltene Verbindung der Formel V in eine andere Verbindung der Erfindung, z.B. eine solche, worin die freie Hydroxygruppe verestert ist und/oder worin $R_2$ eine andere Bedeutung als Wasserstoff hat; wobei man gewünschtenfalls reaktionsfähige funktionelle Gruppen intermediär bei der Ausführung eines der genannten Verfahren schützt und anschliessend die freie Verbindung isoliert, und/oder eine erhaltene Verbindung der Formel I in eine andere Verbindung der Formel I umwandelt, und/oder eine erhaltene freie Verbindung in ein Salz oder ein Salz in die freie Verbindung oder in ein anderes Salz umwandelt; und/oder ein erhaltenes Gemisch von Isomeren oder Racematen in die einzelnen Isomere oder Racemate auftrennt, und/oder ein Racemat in die optischen Antipoden aufspaltet.

19. Verbindungen der Formel III

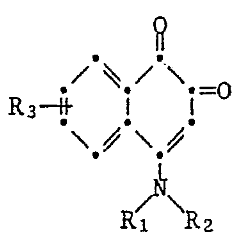

(III)

worin $R_1$ unsubstituiertes oder durch Niederalkyl substituiertes $C_3$-$C_7$-Cycloalkyl, unsubstituiertes oder durch Niederalkyl substituiertes $C_7$-oder $C_8$-Bicycloalkyl, unsubstituiertes oder durch Niederalkyl substituiertes Adamantyl, 4-Piperidinyl oder N-Niederalkyl oder Aryl-niederalkyl substituiertes Piperidinyl, 1- oder 2-Indanyl, 1- oder 2-Tetrahydronaphthyl, 1- oder 2-Perhydroindanyl, 1- oder 2-Perhydronaphthyl bedeutet; $R_2$ Wasserstoff, Niederalkyl, $C_3$-$C_7$-Cycloalkyl, Aryl-niederalkyl, $C_3$-$C_7$-Cycloalkyl-niederalkyl, Niederalkanoyl oder Aryl-niederalkanoyl darstellt; oder $R_1$ und $R_2$, zusammen mit dem Stickstoffatom, an das sie gebunden sind, für Pyrrolidino, Piperidino, Perhydroazepino, Morpholino, Thiomorpholino, Tetrahydro- oder Perhydro-(isochinolinyl oder chinolinyl), Dihydro- oder Perhydro-indolyl, Piperazino oder N-Niederalkyl-piperazino stehen; $R_3$ Wasserstoff, Niederalkyl, Halogen oder Niederalkoxy bedeutet; pharmazeutisch verwendbare Säureadditionssalze von diesen Verbindungen und pharmazeutische Präparate enthaltend solche Verbindungen der Formel III oder entsprechende Salze davon zusammen mit einem oder mehreren pharmazeutisch annehmbaren Trägermaterialien, als 5-Lipoxygenase-Hemmer.

20. Verbindungen der Formel III, worin $R_1$ $C_5$-$C_7$-Cycloalkyl, Bicyclo[2.2.1]heptyl, Adamantyl, 1- oder 2-Indanyl oder Perhydroindanyl, oder 1- oder 2-Tetrahydro- oder Perhydronaphthyl bedeutet, $R_2$

Wasserstoff, Niederalkyl, Aryniederalkyl, $C_5$-$C_7$-Cycloalkyl-niederalkyl, Niederalkanoyl oder Arylniederalkanoyl bedeutet; oder $R_1$ und $R_2$ zusammen mit dem Stickstoffatom, an welches sie gebunden sind, für Pyrrolidino, Piperidino, Perhydroazepino, Morpholino, Thimorpholino, Piperazino oder N-Niederalkyl-piperazino, Tetrahydro- oder Perhydro-isochinolinyl, Tetrahydro-oder Perhydrochinolinyl, Dihydro-oder Perhydroindolyl stehen; $R_3$ Wasserstoff, Niederalkyl oder Halogen bedeutet; pharmazeutisch verwendbare Säureadditionssalze von diesen Verbindungen und pharmazeutische Präparate enthaltend solche Verbindungen der Formel III oder entsprechende Salze davon zusammen mit einem oder mehreren pharmazeutisch annehmbaren Trägermaterialien, als 5-Lipoxygenase-Hemmer.

21. Verbindungen der Formel III, worin $R_1$ Cyclopentyl, Cyclohexyl, Cycloheptyl, 2-Norbornyl, 1- oder 2-Adamantyl, 1-oder 2-Indanyl, oder 1-oder 2-Perhydroindanyl bedeutet; $R_2$ Wasserstoff, $C_1$-$C_4$-Alkyl oder Aryl-$C_1$-$C_4$-alkyl bedeutet; und $R_3$ Wasserstoff ist, pharmazeutisch verwendbare Säureadditionssalze von diesen Verbindungen und pharmazeutische Präparate enthaltend solche Verbindungen der Formel III oder entsprechende Salze davon zusammen mit einem oder mehreren pharmazeutisch annehmbaren Trägermaterialien, als 5-Lipoxygenase-Hemmer.

22. Verbindungen der Formel III, worin $R_1$ und $R_2$ zusammen mit dem Stickstoffatom, an welches sie gebunden sind, für Tetrahydro- oder Perhydro-isochinolinyl, Tetrahydro- oder Perhydrochinolinyl, Dihydro-oder Perhydroindolyl stehen; und $R_3$ Wasserstoff bedeutet, pharmazeutisch verwendbare Säureadditionssalze von diesen Verbindungen und pharmazeutische Präparate enthaltend solche Verbindungen der Formel III oder entsprechende Salze davon zusammen mit einem oder mehreren pharmazeutisch annehmbaren Trägermaterialien, als 5-Lipoxygenase-Hemmer.

23. 4-(N-Methyl-cyclohexylamino)-1,2-naphthochinon und pharmazeutisch verwendbare Säureadditionssalze dieser Verbindung und pharmazeutische Präparate enthaltend diese Verbindung oder entsprechende Salze davon zusammen mit einem oder mehreren pharmazeutisch annehmbaren Trägermaterialien, als 5-Lipoxygenase-Hemmer.

24. Verfahren zur Herstellung von den in Anspruch 19 genannten Verbindungen der Formel III, in der alle Symbole die in Anspruch 19 angegebenen Bedeutungen haben, und deren Salzen, gekennzeichnet durch Umsetzung von 1,2-Naphthochinon-Derivaten der Formel VI

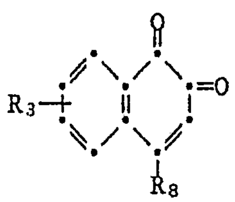

(VI)

worin $R_8$ eine Abgangsgruppe darstellt, mit einem Amin der Formel VII

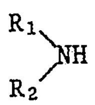

(VII)

worin $R_1$ und $R_2$ unabhängig voneinander oder zusammen die obengenannte Bedeutung haben.